# EUROPÄISCHE PATENTSCHRIFT

(11) **EP 3 569 134 B1**
(45) Veröffentlichungstag und Bekanntmachung des Hinweises auf die Patenterteilung: **21.06.2023**
(21) Anmeldenummer: 19174579.3
(22) Anmeldetag: 15.05.2019
(51) Int. Cl.: A61B 90/70, D06F 39/00, A47L 15/42, A47L 15/48, D06F 37/26, D06F 39/08

(54) **REINIGUNGS- UND/ODER DESINFEKTIONSAUTOMAT**
CLEANING AND/OR DISINFECTION MACHINE
AUTOMATE DE NETTOYAGE ET DE DÉSINFECTION

(30) Priorität: 17.05.2018 DE 102018111904
(43) Veröffentlichungstag der Anmeldung: 20.11.2019
(73) Patentinhaber: Miele & Cie. KG, 33332 Gütersloh (DE)
(72) Erfinder: Bredenbröker, Daniel, 33699 Bielefeld (DE); Schröder, Frank, 32139 Spenge (DE)

(56) Entgegenhaltungen:
- EP-B1- 2 501 849
- WO-A1-2009/023466
- DE-B3-102007 023 020
- US-A1- 2014 010 675
- US-B2- 7 665 332

## Beschreibung

Die Erfindung betrifft einen Reinigungs- und/oder Desinfektionsautomaten, insbesondere eine Wasch- und/oder Spülmaschine, mit einem einen Spülraum bereitstellenden Spülbehälter, der eine Spülbehälterwandung mit einer Öffnung zur Beschickung des Spülraums mit einem Fluid aufweist.

Reinigungs- und/oder Desinfektionsautomaten der gattungsgemäßen Art sind aus dem Stand der Technik an sich gut bekannt, weshalb es eines gesonderten druckschriftlichen Nachweises an dieser Stelle nicht bedarf.

Ein gattungsgemäßer Reinigungs- und/oder Desinfektionsautomat verfügt über einen Spülbehälter, der einen Spülraum bereitstellt. Im bestimmungsgemäßen Verwendungsfall dient der Spülraum der Aufnahme von zu reinigenden und/oder zu desinfizierenden Spülgütern, beispielsweise medizinischen Instrumenten.

Der Spülbehälter weist eine Spülbehälterwandung mit einer Öffnung zur Beschickung des Spülraums mit Fluid auf. Im bestimmungsgemäßen Verwendungsfall dient diese Öffnung in der Spülbehälterwandung dazu, dem Spülraum eine Spülflüssigkeit wie zum Beispiel Wasser zuführen zu können. Diese Öffnung ist bei vorbekannten Automaten typischerweise in einer seitlichen Wandung des Spülbehälters ausgebildet.

Zusätzlich zur Öffnung zur Beschickung des Spülraums mit Spülflüssigkeit ist die Spülbehälterwandung eines aus dem Stand der Technik vorbekannten Reinigungs- und/oder Desinfektionsautomaten mit einer Öffnung zur Abluftabsaugung ausgerüstet. Diese zweite Öffnung in der Spülbehälterwandung dient dazu, nach Abschluss eines bestimmungsgemäß durchlaufenen Reinigungs- und/oder Desinfektionsprogramms im Spülbehälter befindliche Abluft absaugen zu können. Dabei dient eine solche Abluftabsaugung insbesondere der Trocknung von im Spülraum befindlichen Spülgütern.

Die Abluft ist typischerweise aufgrund einer vorangegangenen Desinfektion aufgewärmt und kann zur Wärmerückgewinnung nach einem Abführen aus dem Spülbehälter einem Wärmetauscher zugeführt werden, der der Voraufwärmung einer über die andere Öffnung in der Spülbehälterwandung dem Spülbehälter zuzuführende Spülflüssigkeit dient. Insbesondere aus thermischen Gründen ist die der Abluftabsaugung dienende Spülbehälteröffnung in einem in Höhenrichtung oberen Abschnitt der Spülbehälterwandung ausgebildet.

Die US 7665332 B2 offenbart ein Wäschebehandlungsgerät mit einer Waschtrommel und einem Reinigungsmittelbehälter. Über einen Wassereinlass kann Frischwasser in den Reinigungsmittelbehälter eingeleitet und von dort über eine Wasserleitung in die Waschtrommel eingelassen werden. Der Reinigungsmittelbehälter weist darüber hinaus einen Dampfeinlass auf, durch den Dampf aus der Waschtrommel über eine Dampfleitung in den Reinigungsmittelbehälter gelangen kann.

Die EP 2501849 B1 offenbart ein Wäschepflegegerät mit einem Dampferzeuger, der einen beheizbaren Tank sowie einen Wassereinlass zur Einleitung von Wasser in den Tank und einen Dampfauslass zur Förderung des im Tank erzeugten Dampfes in eine Waschtrommel aufweist.

Die WO 2009023466 A1 offenbart einen Geschirrspüler mit einem Dampferzeuger, der einen Frischwassereinlass zur Einleitung von Wasser und einen Dampfauslass zur Einleitung von Dampf aus dem Dampferzeuger in den Spülbehälter aufweist.

Die DE 102007023020 B3 offenbart eine Wäschebehandlungsmaschine mit einem Einspülkasten und einem Dampferzeuger. Vom Einspülkasten aus wird Frischwasser über eine Wasserleitung in die Waschtrommel geleitet. Im Dampferzeuger erzeugter Dampf wird über eine Dampfleitung in die Waschtrommel eingeblasen.

Die US 2014/010675 A1 offenbart ein Sterilisationsgerät mit einem Sterilisationskompartment zur Aufnahme eines Endoskops und einer Pumpe, mit der Sterilisationsfluid in ein an einem ersten Anschluss angeschlossenes Endoskop eingeleitet werden kann und mit der über einen zweiten Anschluss Sterilisationsfluid aus dem Sterilisationskompartment abgepumpt werden kann.

Obgleich sich die vorbeschriebene Konstruktion des gattungsgemäßen Reinigungs- und/oder Desinfektionsautomaten im alltäglichen Praxiseinsatz bewährt hat, besteht Verbesserungsbedarf, insbesondere mit Blick auf eine vereinfachte konstruktive Ausgestaltung. Es ist deshalb die **Aufgabe** der Erfindung, einen Reinigungs- und/oder Desinfektionsautomaten der gattungsgemäßen Art dahingehend weiterzuentwickeln, dass ein konstruktiv vereinfachter Aufbau bei einer gleichzeitig vereinfachten Handhabung im Anwendungsfall erreicht ist.

Zur **Lösung** dieser Aufgabe wird mit der Erfindung ein Reinigungs- und/oder Desinfektionsautomat der eingangs genannten Art mit den Merkmalen von Anspruch 1 vorgeschlagen.

Die erfindungsgemäße Ausgestaltung sieht eine Fluiddurchströmungseinrichtung vor, die dem Anschluss sowohl an eine Fluidzuführung als auch an eine Fluidabführung dient. Dabei wirkt die Fluiddurchströmungseinrichtung mit der in der Spülbehälterwandung ausgebildeten Öffnung strömungstechnisch zusammen. Gemäß der erfindungsgemäßen Konstruktion ist mithin nur eine Öffnung in der Spülbehälterwandung vorgesehen, wobei diese Öffnung nicht nur dazu dient, eine Beschickung des Spülraums mit Spülflüssigkeit vornehmen zu können, sondern auch dazu, aus dem Spülraum ein Fluid abführen zu können. Auf eine weitere, d.h. zweite Öffnung ist mithin in Abkehr zum Stand der Technik vollends verzichtet.

Im bestimmungsgemäßen Verwendungsfall wird dem Reinigungs- und/oder Desinfektionsautomaten zur Durchführung eines Spülprogramms Wasser als Spülflüssigkeit zugeführt. Dies geschieht mittels der Fluiddurchströmungseinrichtung, zu welchem Zweck diese eine erste Anschlussvorrichtung aufweist. Das als Spülflüssigkeit dienende Wasser wird über die Fluiddurchströmungseinrichtung und die in der Spülbehälterwandung ausgebildete Öffnung in den Spülraum geleitet.

Eine Absaugung insbesondere von Dampf im Nachgang einer bestimmungsgemäß durchgeführten Reinigung und/oder Desinfektion findet durch die gleiche in der Spülbehälterwandung ausgebildete Öffnung hindurch statt. Es ist mithin für den Wassereinlauf einerseits und die Abluftabsaugung andererseits ein und dieselbe Öffnung in der Spülbehälterwandung vorgesehen. Dabei erfolgt die Abluftabsaugung über die zweite von der Fluiddurchströmungseinrichtung bereitgestellte Anschlussvorrichtung.

Die erfindungsgemäße Ausgestaltung erweist sich aus mehreren Gründen als vorteilhaft. Zum einen bedarf es nur einer Öffnung in der Spülbehälterwandung für eine Fluidzufuhr einerseits und eine Fluidabfuhr andererseits. Dies ist im Unterschied zum Stand der Technik konstruktiv einfacher und im Betrieb wartungsfreundlicher. Dabei gestaltet sich die Installation der erfindungsgemäß vorgesehenen Fluiddurchströmungseinrichtung einfach, da diese lediglich auf die Öffnung in der Spülbehälterwandung aufzusetzen und mit der Spülbehälterwandung zu verbinden, beispielsweise zu verschrauben ist. Die Fluiddurchströmungseinrichtung kann mithin verwendungsfertig vorgefertigt und alsdann in einfacher Weise für eine bestimmungsgemäße Verwendung am Spülbehälter montiert werden.

Die nach der Erfindung vorgesehene Fluiddurchströmungseinrichtung ist in vorteilhafter Weise wartungsfrei. Es entfallen insbesondere die hinsichtlich einer vorbekannten Vorrichtung zur Wasserzuführung in regelmäßigen Intervallen durchzuführenden Spül- und Reinigungsvorgänge. Dies deshalb, weil eine Warmluftabfuhr zwecks Trocknung von im Spülraum befindlichen Spülgütern auch zu einer Trocknung der Fluiddurchströmungseinrichtung führt, so dass ungewünschten Wasseran- und/oder -ablagerungen wirkungsvoll vorgebeugt ist.

Nicht zuletzt erweist sich das erfindungsgemäße System gegenüber dem Stand der Technik, wonach zwei voneinander getrennte Systeme vorgesehen sind, als weniger kostgenintensiv in der Herstellung und weniger aufwendig in der späteren Wartung.

Es ist gemäß einem weiteren Merkmal der Erfindung vorgesehen, dass die erste Anschlussvorrichtung einer Zufuhr eines flüssigen Fluides, insbesondere einer Zufuhr von Wasser dient, wobei eine Mehrzahl von Einzelanschlüssen vorgesehen ist. Die erste Anschlussvorrichtung verfügt über eine Mehrzahl von Einzelanschlüssen, beispielsweise über vier Einzelanschlüsse, wobei jeder Einzelanschluss dazu dient, an eine Wasserzuführungsleitung angeschlossen zu werden. Die Unterteilung in Einzelanschlüsse ist bevorzugt, um dem Spülraum Wasser aus unterschiedlichen Quellen gleichsam zuführen zu können. So kann beispielsweise ein Einzelanschluss für die Zuführung von Leitungswasser, ein Einzelanschluss für die Zuführung von erwärmtem Wasser sowie zwei Einzelanschlüsse für die jeweilige Zuführung von vollentsalztem Wasser vorgesehen sein. Das Vorsehen von zwei Einzelanschlüssen für die jeweilige Zuführung von vollentsalztem Wasser ist bevorzugt, um eine größere Menge an Wasser pro Zeiteinheit zuführen zu können. Bevorzugter Weise ist die erste Anschlussvorrichtung konstruktiv so ausgelegt, dass über die Einzelanschlüsse eine Zuflussmenge je Einzelanschluss von ca. 14 l/min ermöglicht ist.

Gemäß einem weiteren Merkmal der Erfindung ist vorgesehen, dass die zweite Anschlussvorrichtung einer Entnahme eines gasförmigen Fluides, insbesondere eines Luftgemisches wie beispielsweise Dampf dient. Demnach erfolgt die Abluftabsaugung über einen zu den Einzelanschlüssen für die Wasserzuführung separaten Kanal. Dies gestattet eine optimierte Anschlussauslegung für die Wasserzuführung einerseits und die Abluftabführung andererseits.

Gemäß einem weiteren bevorzugten Merkmal der Erfindung ist vorgesehen, dass die Fluiddurchströmungseinrichtung ein kreiszylinderförmiges Gehäuse aufweist, dessen spülbehälterseitige Stirnseite für einen strömungstechnischen Anschluss an die Öffnung in der Spülbehälterwandung zumindest teilweise offen ausgebildet ist.

Diese Ausgestaltung erweist sich konstruktiv als sehr einfach. So verfügt die Fluiddurchströmungseinrichtung über ein Gehäuse. Dieses stellt die Anschlüsse für die Wasserzufuhr bzw. die Abluftabfuhr bereit. Dabei ist das Gehäuse bevorzugter Weise kreiszylinderförmig ausgebildet, weist also im Querschnitt eine kreisförmige Ausgestaltung auf.

Die Anschlüsse für die Wasserzufuhr bzw. die Abluftabfuhr sind an einer Stirnseite des Gehäuses ausgebildet. Die andere Stirnseite des Gehäuses dient dem strömungstechnischen Anschluss des von der Gehäusewandung umschlossenen Volumenraums an die in der Spülbehälterwandung ausgebildete Öffnung. Zu diesem Zweck ist die spülbehälterseitige Stirnwand des Gehäuses zumindest teilweise offen ausgebildet. Im endmontierten Zustand ist das Gehäuse fluiddicht an die Spülbehälterwandung angeflanscht, wobei die Gehäusewandung die in der Spülbehälterwandung ausgebildete Öffnung umschließt. Es ist so eine strömungstechnische Verbindung zwischen dem Spülraum und dem vom Gehäuse der Fluiddurchströmungseinrichtung umschlossenen Volumenraum ausgebildet, und zwar unter Zwischenordnung der in der Spülbehälterwandung ausgebildeten Öffnung.

Es ist gemäß einem weiteren Merkmal vorgesehen, dass das Gehäuse einen mit der Öffnung zusammenwirkenden Spritzschutz aufweist, der in Höhenrichtung oberhalb der Öffnung angeordnet ist.

Dieser Spritzschutz dient dazu, dass im Inneren des Spülbehälters im bestimmungsgemäßen Verwendungsfall umgewälzte Spülflüssigkeit nicht auf direktem Wege durch die in der Spülbehälterwandung ausgebildete Öffnung hindurch in den vom Gehäuse der Fluiddurchströmungseinrichtung umgebenen Volumenraum einströmen kann. Es ist insofern eine Labyrinthführung gegeben, an der unter Umständen aus dem Spülbehälter hochspritzende Spülflüssigkeit abprallt, womit die kinetische Energie der hochgespritzten Spülflüssigkeit nicht mehr ausreicht, um zu den Anschlüssen der Fluiddurchströmungseinrichtung für die Wasserzufuhr einerseits und die Abluftabfuhr andererseits gelangen zu können.

Es ist gemäß einem weiteren Merkmal der Erfindung vorgesehen, dass das Gehäuse ein mit der ersten Anschlussvorrichtung zusammenwirkendes Fluidleitelement aufweist, das nach Art eines Trichters ausgebildet ist und das sich ausgehend von der ersten Anschlussvorrichtung in Richtung auf die spülbehälterseitige Öffnung konisch verjüngt.

Dieses nach Art eines Trichters ausgebildete Fluidleitelement dient dazu, über die erste Anschlussvorrichtung aufgegebenes Wasser kanalisiert in den Spülraum einzuleiten. Eine Zufuhr von Wasser in den Spülraum des Spülbehälters findet mithin unter strömungstechnischer Zwischenschaltung des Fluidleitelementes statt.

Das Fluidleitelement dient in vorteilhafter Weise der Labyrinthausgestaltung, und zwar für die Strömungswege für Wasser in Zuführrichtung als auch für Abluft in Abführrichtung. Es können damit unkontrollierte Wasser- oder Abluftwege in wirkungsvoller Weise vermieden werden.

Gemäß einem weiteren bevorzugten Merkmal der Erfindung ist vorgesehen, dass die Einzelanschlüsse der ersten Anschlussvorrichtung unter Zwischenschaltung einer jeweiligen Freifallstrecke in das Fluidleitelement einmünden. Es ist demnach keine Verrohrung mit dem Spülbehälter vorgesehen. Die Fluiddurchströmungseinrichtung verfügt vielmehr nur über Einzelanschlüsse, die dem jeweiligen Anschluss einer Wasserzuführungsleitung dienen. Diese Einzelanschlüsse münden unter jeweiliger Zwischenordnung einer Freifallstrecke in das Fluidleitelement ein. Im bestimmungsgemäßen Verwendungsfall strömt Wasser über die jeweils beaufschlagten Einzelanschlüsse mithin frei in das Fluidleitelement ein. Aufgrund der trichterförmigen Ausgestaltung des Fluidleitelementes findet sodann eine Kanalisierung statt und das Wasser tritt spülbehälterseitig aus dem Fluidleitelement aus, von wo aus es dann in den Spülbehälter gelangt.

Die vorbeschriebene Ausgestaltung ist insbesondere aus zwei Gründen von Vorteil. Zum einen ist wirkungsvoll unterbunden, dass etwaiges Spritzwasser zurück durch die Fluiddurchströmungseinrichtung bis hin zu den Einzelanschlüssen spritzen kann. Ferner ist sichergestellt, dass auch im Falle einer ungewollten Überflutung des Spülraums kein Rückfluss in die Einzelanschlüsse stattfinden kann. Es sind zu diesem Zweck im Weiteren noch näher beschriebene Fenster in der Wandung des Gehäuses der Fluiddurchströmungseinrichtung vorgesehen, durch die hindurch im Überflutungsfall Spülflüssigkeit abgeführt werden kann.

Es ist gemäß einem weiteren bevorzugten Merkmal der Erfindung vorgesehen, dass der Spritzschutz ein mit einer Mittenausnehmung ausgebildetes Scheibenelement ist, wobei das Fluidleitelement mit seinem spülbehälterseitigen Endabschnitt die Mittenausnehmung durchragt.

Gemäß dieser bevorzugten Ausführungsform der Erfindung ist der Spritzschutz ein mit einer Mittenausnehmung ausgerüstetes Scheibenelement. Dieses Scheibenelement ist in Höhenrichtung oberhalb der Öffnung in der Spülbehälterwandung angeordnet. Es hält insbesondere aus dem Spülbehälter spritzende Spülflüssigkeit zurück. Zwecks Zufuhr von Spülflüssigkeit ist die in dem Scheibenelement ausgebildete Mittenausnehmung vorgesehen. Durch diese hindurch ragt das trichterförmig ausgebildete Fluidleitelement mit seinem spülbehälterseitigen Endabschnitt. Aufgabeseitig in das Fluidleitelement eingebrachtes Wasser gelangt mithin mittels des Fluidleitelementes geführt durch die im Spritzschutz ausgebildete Mittenausnehmung hindurch, von wo aus dann ein Einströmen in den Spülbehälter durch die dafür vorgesehene Öffnung stattfinden kann.

Es ist gemäß einem weiteren bevorzugten Merkmal der Erfindung vorgesehen, dass die Öffnung in der Spülbehälterwandung ringförmig ausgebildet ist und die Mittenöffnung des Spritzschutzes umfangsseitig umgibt.

Gemäß dieser Ausführungsform der Erfindung ist die in der Spülbehälterwandung vorgesehene Öffnung nicht als zentrische Ausnehmung ausgebildet. Die Öffnung ist vielmehr ringförmig ausgebildet, ggf. durch Stege unterbrochen, so dass eine ringförmige Segmentanordnung gegeben ist. In Höhenrichtung oberhalb dieser ringförmig ausgebildeten Ausnehmung ist das als Spritzschutz dienende Scheibenelement positioniert. Das Scheibenelement ist mithin direkt oberhalb der ringförmigen Öffnung ausgebildet, womit ein ungehinderter Durchfluss von aus dem Spülraum stammender Spülflüssigkeit in den Volumenraum des Gehäuses der Fluiddurchströmungseinrichtung oberhalb des Spritzschutzes unterbunden ist.

Es ist gemäß einem weiteren bevorzugten Merkmal der Erfindung vorgesehen, dass das trichterförmig ausgebildete Fluidleitelement unter Belassung eines Ringraums beabstandet zur Gehäusewandung des Gehäuses der Fluiddurchströmungseinrichtung angeordnet ist.

Diese Beabstandung schafft den Durchströmungsraum insbesondere im Falle abzusaugender Abluft. Denn im bestimmungsgemäßen Absaugfall durchströmt die in der Spülbehälterwandung ausgebildete Öffnung Abluft, die dann die daran strömungstechnisch angeschlossene Fluiddurchströmungseinrichtung durchströmt, wobei das trichterförmig ausgebildete Fluidleitelement sowohl durchströmt als auch durch den belassenen Ringraum außenseitig passiert wird. Damit ist eine insgesamt hinreichend große Querschnittsfläche geschaffen, um im bestimmungsgemäßen Verwendungsfall hinreichende Mengen an Abluft absaugen zu können.

Es ist gemäß einem weiteren bevorzugten Merkmal der Erfindung vorgesehen, dass in Höhenrichtung oberhalb des Ringraums ein den Ringraum abdeckendes Ringteil angeordnet ist. Dieses Ringteil dient dazu, im Abluftfall abgesogene Abluft in Richtung des dafür vorgesehenen Abluftstutzens umzulenken. Des Weiteren dient dieses Ringteil dazu, Wasser im Zuführfall daran zu hindern, außerhalb des Fluidleitelementes an diesem vorbeizuströmen. Insofern dient auch dieses Ringteil zur Ausbildung der schon mit der übrigen Baukomponentenanordnung geschaffenen Labyrinthführung.

Gemäß einem weiteren bevorzugten Merkmal der Erfindung ist vorgesehen, dass in Höhenrichtung unterhalb der Einlassstutzen der ersten Anschlussvorrichtung Fenster in der Gehäusewandung ausgebildet sind.

Diese Fenster dienen in vorteilhafter Weise zwei Zwecken. Zum einen dienen sie als Überlaufschutz und zum anderen sorgen sie für eine hinreichende Zufuhr von Frischluft bei einem zu starken Abluftsog.

Es ist in jedem Fall zu verhindern, dass über die Einzelanschlüsse Spülflüssigkeit zurück in die daran angeschlossenen Rohrleitungen strömen kann. Aus diesem Grunde ist zum einen die schon vorbeschriebene Freifallstrecke je Einzelanschluss vorgesehen. Kommt es indes zu einer Überflutung der Fluiddurchströmungseinrichtung, beispielsweise durch Verstopfung der spülbehälterseitigen Öffnung, so hilft auch dieses Freifallstrecke nicht, um ein Zurückströmen von Spülflüssigkeit in die Einzelanschlüsse sicher vermeiden zu können. Aus diesem Grunde verfügt die Wandung des Gehäuses der Fluiddurchströmungseinrichtung über Ausnehmungen in Form von Fenstern, die in Höhenrichtung unterhalb der Einzelanschlüsse ausgebildet sind. Im Überflutungsfall kann über diese Fenster Spülflüssigkeit aus dem Gehäuse der Fluiddurchströmungseinrichtung abströmen, und zwar bevor die aufgestaute Spülflüssigkeit die Einzelanschlüsse erreicht.

Im bestimmungsgemäßen Trocknungsfall wird über die zweite Anschlussvorrichtung Abluft abgesaugt. Je nach eingesetztem Abluftaggregat wird ein bestimmtes Abluftvolumen je Zeiteinheit abgesaugt. Dabei kann sich in nachteiliger Weise eine zu große Abluftmenge einstellen, was dann einen nachteiligen Einfluss auf den eigentlich gewünschten Trocknungsprozess hat. Um diesen Nachteil zu vermeiden und eine Fluiddurchströmungseinrichtung bereitzustellen, die unabhängig von dem angeschlossenen Abluftaggregat zuverlässig und wirkungsvoll ist, sind die in der Gehäusewandung vorgesehenen Fenster ausgebildet. Über diese kann nämlich im Falle eines zu hoch eingestellten Abluftstroms Frischluft von außerhalb des Spülbehälters angesogen werden, was verhindert, dass eine zu große Menge an Abluft aus dem Spülbehälter abgesogen wird.

Die vorgesehenen Fenster dienen damit nicht nur einem Überflutungsschutz, sie tragen auch dafür Sorge, dass im Abluftfall Frischluft in den Abluftstrom mit eingemischt werden kann, so dass eine zu große Mengenabfuhr an Abluft unterbunden ist.

Gemäß einem weiteren bevorzugten Merkmal der Erfindung ist vorgesehen, dass die zweite Anschlussvorrichtung einen Anschlussstutzen aufweist, der koaxial zum Fluidleitelement ausgerichtet ist. Es ist so eine strömungstechnisch direkte Verbindung zwischen Fluidleitelement und Anschlussstutzen der Anschlussvorrichtung gegeben, was strömungstechnisch von Vorteil ist. Dabei kann in vorbeschriebener Weise das Fluidleitelement auch außenseitig von Abluft umströmt werden, zu welchem Zweck das Fluidleitelement unter Zwischenordnung eines Ringraums beabstandet zur Wandung des Gehäuses der Fluiddurchströmungseinrichtung angeordnet ist.

Gemäß einem weiteren bevorzugten Merkmal der Erfindung ist vorgesehen, dass der Anschlussstutzen innenseitig eine umlaufende Rinne aufweist. Diese Rinne dient dazu, etwaiges Kondensat, das sich aus der Abluft innenseitig des Anschlussstutzens niederschlägt, abzufangen. Es ist damit ein unkontrollierter Rückfluss von Kondensat in den Spülbehälter vermieden.

Gemäß einem weiteren bevorzugten Merkmal der Erfindung ist vorgesehen, dass der von der Rinne bereitgestellte Volumenraum an eine Ablauföffnung strömungstechnisch angeschlossen ist. Diese Ablauföffnung stellt sicher, dass etwaiges von der Rinne aufgefangenes Kondensat nach außen, d.h. aus dem Gehäuse der Fluiddurchströmungseinrichtung geführt werden kann. Zu diesem Zweck kann ergänzend ein Röhrchen vorgesehen sein, das an die Ablauföffnung strömungstechnisch angeschlossen ist.

Mit der erfindungsgemäßen Ausgestaltung wird insgesamt eine Konstruktion vorgeschlagen, die den Anforderungen nach DIN EN 1717 gerecht wird. Dabei erfolgt in Abkehr zum Stand der Technik über nur eine einzige Öffnung in der Spülbehälterwandung sowohl eine Wasserzufuhr als auch eine Abluftabfuhr.

Die im Inneren des Gehäuses der Fluiddurchströmungseinrichtung angeordneten Bauteile, d.h. der Spritzschutz, das Fluidleitelement sowie das Ringteil sind derart aufeinander abgestimmt und in ihrer jeweiligen Relation derart zueinander angeordnet, dass eine Labyrinthführung sowohl für zuströmendes Wasser als auch für abströmende Abluft gegeben ist. Damit sind insbesondere ungewollte Fluidrückführungen in die erste Anschlussvorrichtung sicher unterbunden.

So wird ein Herausspritzen von Spülflüssigkeit durch die in der Gehäusewandung ausgebildeten Fenster hindurch während eines Wassereinlaufs dadurch verhindert, dass oberhalb des kegelförmig ausgebildeten Fluidleitelementes ein Ringteil angeordnet ist. Beim Bestromen eines oder mehrerer Einzelanschlüsse trifft der davon abgegebene Wasserstrahl zuerst auf das als Kegelstutzen ausgebildete Fluidleitelement und das Wasser wird von diesem nach unten abgeleitet. Durch einen abgerundeten Auslauf am spülbehälterseitigen Ende des Kegelstutzens fließt das Wasser auf den Spülraum, d.h. die Spülbehälterwandung. Über die ringförmige Öffnung in der Spülbehälterwandung fließt das Wasser sodann in den Spülraum und füllt diesen bis zur gewünschten Menge.

Die Fluiddurchströmungseinrichtung ist bevorzugter Weise in Höhenrichtung oben am Spülbehälter angeordnet, d.h. auf diesen aufgesetzt. Dabei besitzt die obere Spülraumwandung eine Neigung von ca. 8° zur Horizontalen, und zwar in Richtung nach außen. So wird ein Aufstauen von Wasser zwischen Spülraumseite und Fluiddurchströmungseinrichtung verhindert. Von der Fluiddurchströmungseinrichtung abgegebenes Wasser fließt vielmehr der Schwerkraft folgend der Spülraumwandung in Entsprechung der Neigung entlang, bis es zur ringförmigen Öffnung in der Spülraumwandung gelangt, durch die hindurch es dann in den Spülraum gelangt. Die erfindungsgemäße Fluiddurchströmungseinrichtung wird trotz der Neigung der Spülraumwandung senkrecht montiert, was dadurch erreicht ist, dass die spülbehälterseitige Stirnseite des Gehäuses der Fluiddurchströmungseinrichtung eine entsprechende Gegenneigung aufweist.

Nach DIN EN 1717 ist verlangt, dass sich Wasser beim Einlaufvorgang nicht bis zu den Einzelanschlüssen zurückstauen darf, falls Öffnungen in der Spülraumoberseite zum Beispiel versperrt sind. Eine mögliche Rücksaugung bzw. ein möglicher Rückfluss von verunreinigter Spülflüssigkeit ins öffentliche Leitungsnetz muss in jedem Fall verhindert sein. Dies wird bei der erfindungsgemäßen Ausgestaltung durch die in der Gehäusewandung des Gehäuses der Fluiddurchströmungseinrichtung ausgebildeten Fenster realisiert. Diese bilden einen freien Auslauf gemäß Typ AB nach DIN EN 1717. Trotz einer möglichen Zulaufmenge von 48 l/min wird bei einem Rückstau DIN EN 1717 konform über diese Fenster eine Entwässerung erreicht.

Nach DIN EN 1717 ist ferner verlangt, dass beim Einlaufvorgang kein Wasser in die Einzelanschlüsse zurückspritzen darf. Dies wird bei der erfindungsgemäßen Ausgestaltung wirksam durch die Form des kegelförmig ausgebildeten Fluidleitelementes erreicht.

Dass Spülflüssigkeit während eines bestimmungsgemäßen Spül- und Umwälzprozesses nicht über die Fenster herausspritzen kann, wird durch die Schaffung der schon vorbeschriebenen Labyrinthführung im Inneren des Gehäuses der Fluiddurchströmungseinrichtung gewährleistet. Wasser, welches über die ringförmige Öffnung in der Spülraumwandung heraustritt, trifft zunächst auf den Spritzschutz. Sollte an diesem Spritzschutz vorbei Wasser in den weiteren Innenraum des Gehäuses der Fluiddurchströmungseinrichtung gelangen, so reicht die im Wasser vorhandene kinetische Energie nicht mehr aus, dass das Wasser bis zu den in der Gehäusewandung ausgebildeten Fenstern strömen kann. Darüber hinaus ist die Öffnung in der Spülbehälterwandung so konzipiert, dass Sprühstrahlen von Sprüharmen im Spülraum mit Blechen abgeschattet werden und nicht direkt in das Gehäuse der Fluiddurchströmungseinrichtung gelangen können.

Die Trocknung im Spülraum erfolgt über die Zuführung von Heißluft, welche von Trocknungsaggregaten erzeugt wird. Der im Spülraum entstehende Dampf wird über die zweite Anschlussvorrichtung der Fluiddurchströmungseinrichtung abgesaugt. Zu diesem Zweck ist die Mittenöffnung in dem als Spritzschutz dienenden Scheibenelement so gewählt, dass der Volumenstrom ausreichend groß ist, um den Dampf auch bei niedriger zentraler Absaugleistung abführen zu können. Dabei ist diese Mittenöffnung aber auch nur eben so groß, dass Wasser beim Umwälzen nicht nach oben hinaus spritzen kann.

Ein Teil des im Abluftbetrieb abgesogenen Dampfes strömt außen am Fluidleitelement entlang. Ein über dem Fluidleitelement installiertes Ringteil führt den Dampf in Richtung des Anschlussstutzens der zweiten Anschlussvorrichtung. Für zurücklaufendes Kondensat gibt es in schon vorbeschriebener Weise einen Kondensatauffang.

Die in der Gehäusewandung des Gehäuses der Fluiddurchströmungseinrichtung ausgebildeten Fenster dienen neben der Einhaltung der DIN EN 1717 auch der Nebenluftansaugung. So können zentrale Abluftsysteme in der Regel keinen konstanten Abluftstrom gewährleisten. Es können mithin kleinere aber auch wesentlich größere Ansaugleistungen anliegen. Würde eine hohe Absaugleistung ohne Nebenluftansaugmöglichkeit anliegen, so könnte unter Umständen die gewünschte Programmtemperatur innerhalb insbesondere der Trocknungsphase nicht erreicht werden. Aus diesem Grunde sind die Fenster so ausgelegt, dass bei Abluftleistungen von über 250 m³/min trotzdem ein maximal beladener Spülraum innerhalb der gewünschten Zeit auf die erforderlichen 90°C aufgeheizt werden kann.

Weitere Merkmale und Vorteile der Erfindung ergeben sich aus der nachfolgenden Beschreibung anhand der Figuren. Dabei zeigen
- Fig. 1: in schematischer Perspektivansicht eine erfindungsgemäße Fluiddurchströmungseinrichtung;
- Fig. 2: in schematischer Seitenansicht einen Spülbehälter mit daran angeordneter Fluiddurchströmungseinrichtung;
- Fig. 3: in perspektivischer Schnittdarstellung eine erfindungsgemäße Fluiddurchströmungseinrichtung;
- Fig. 4: in geschnittener Seitenansicht eine erfindungsgemäße Fluiddurchströmungseinrichtung;
- Fig. 5: in schematischer Schnittdarstellung eine erfindungsgemäße Fluiddurchströmungseinrichtung im Abluftbetrieb und
- Fig. 6: in schematischer Schnittdarstellung eine erfindungsgemäße Fluiddurchströmungseinrichtung im Wasserzufuhrbetrieb.

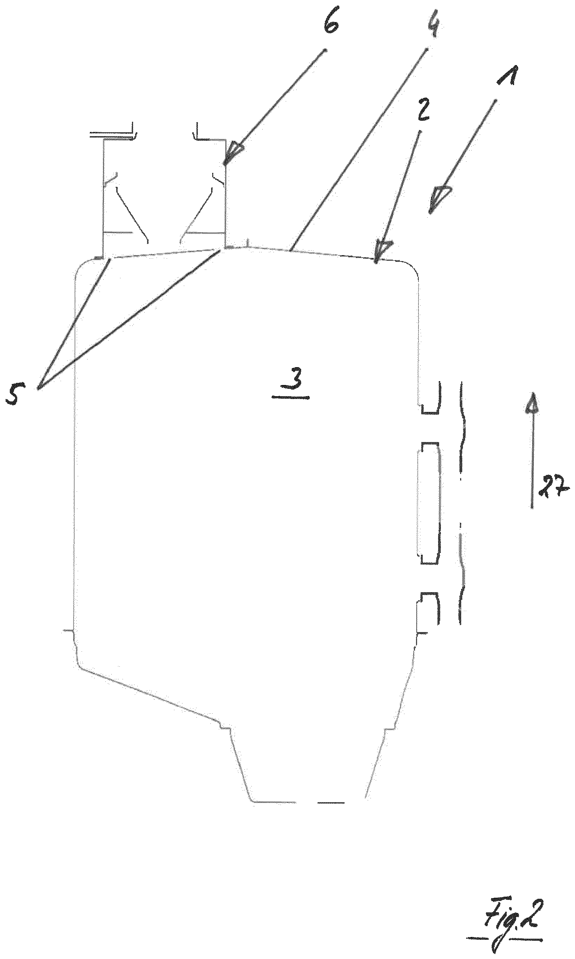

Fig. 2 lässt in rein schematischer Seitenansicht den Spülbehälter 2 eines ansonsten in den Figuren nicht näher dargestellten Reinigungs- und/oder Desinfektionsautomaten 1 erkennen.

Der Spülbehälter 2 stellt einen Spülraum 3 bereit, der im bestimmungsgemäßen Verwendungsfall der Aufnahme von zu reinigendem Spülgut dient.

Der Spülbehälter 2 verfügt über eine Spülbehälterwandung 4 mit einer in Höhenrichtung 27 oberseitig ausgebildeten Öffnung 5. Diese Öffnung 5 ist ringförmig ausgebildet und dient im bestimmungsgemäßen Verwendungsfall des Reinigungs- und/oder Desinfektionsautomaten 1 sowohl der Zufuhr von als Spülflüssigkeit dienendem Wasser als auch der Abfuhr von Abluft, insbesondere Dampf.

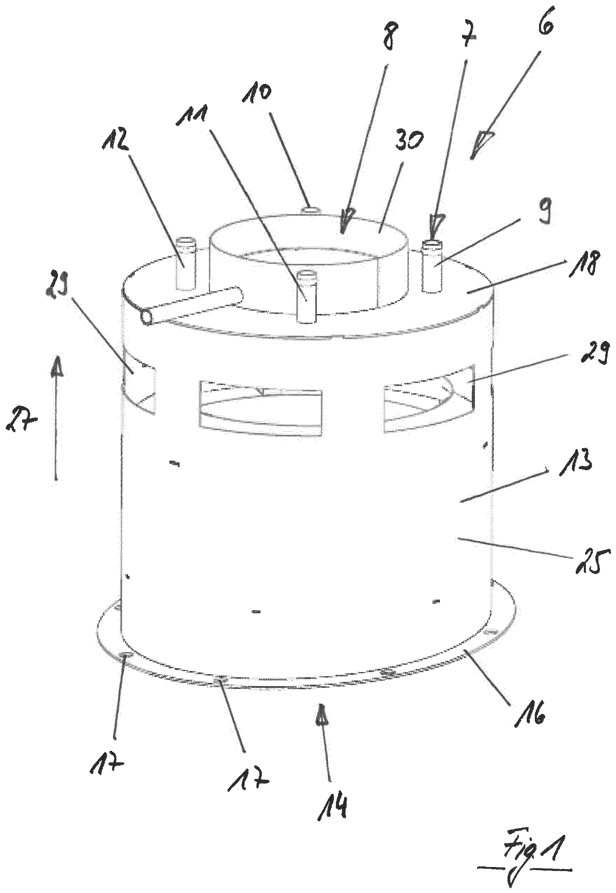

Die in der Spülbehälterwandung 4 ausgebildete Öffnung 5 steht in strömungstechnischer Verbindung mit einer Fluiddurchströmungseinrichtung 6, die eine erste Anschlussvorrichtung 7 für eine Fluidzufuhr und eine zweite Anschlussvorrichtung 8 für eine Fluidabfuhr aufweist. Der Aufbau dieser Fluiddurchströmungseinrichtung 6 ergibt sich insbesondere aus einer Zusammenschau der Figuren 1 und 3 bis 4.

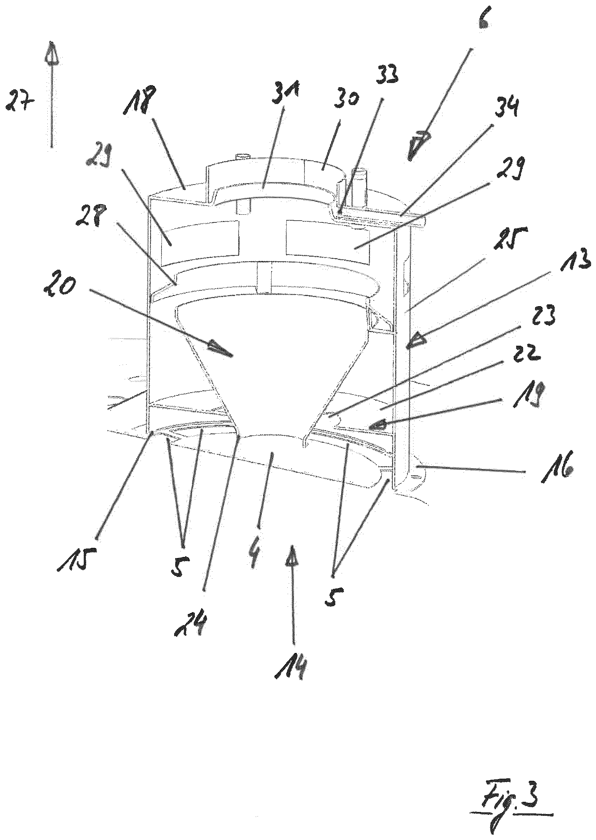

Die Fluiddurchströmungseinrichtung 6 verfügt, wie dies insbesondere die Darstellung nach Fig. 1 erkennen lässt, über ein Gehäuse 13. Dieses Gehäuse 13 ist kreiszylinderförmig ausgebildet und verfügt über eine Gehäusewandung 25. Die in Höhenrichtung 27 obere Stirnseite des Gehäuses 13 ist mittels eines Deckels 18 verschlossen. Die in Höhenrichtung 27 untere Stirnseite 14, d.h. die dem Spülbehälter 2 zugewandte Stirnseite 14 ist offen ausgebildet und verfügt über eine dementsprechende Ausnehmung 15, wie sich insbesondere aus der Darstellung nach Fig. 3 ergibt.

Das Gehäuse 13 weist spülraumseitig einen umlaufenden Flansch 16 mit Bohrungen 17 auf. Dieser Flansch 16 dient der Anordnung des Gehäuses 13 an der Spülbehälterwandung 4. Zu diesem Zweck kommen Schrauben zum Einsatz, die die im Flansch 16 ausgebildeten Bohrungen 17 durchgreifen.

Wie sich insbesondere aus der Darstellung nach Fig. 3 ergibt, ist die in der Spülbehälterwandung 4 ausgebildete Öffnung 5 ringförmig ausgebildet. Dabei sind im gezeigten Ausführungsbeispiel einzelne Ringsegmente gegeben, die durch Stege der Spülraumwandung 4 voneinander beabstandet sind. Das Gehäuse 13 ist in seinem Querschnitt so bemessen, dass es auf der Spülbehälterwandung 4 fluiddicht aufsitzt und die ringförmig ausgebildete Öffnung 5 in der Spülbehälterwandung 4 umschließt. Über die die offene Stirnseite 14 ausbildende Ausnehmung 15 ist eine strömungstechnische Verbindung zwischen der in der Spülbehälterwandung 4 ausgebildeten Öffnung 5 und dem vom Gehäuse 13 umgebenen Volumenraum geschaffen.

Der Deckel 18 des Gehäuses 13 trägt die beiden Anschlussvorrichtungen 7 und 8. Dabei verfügt die erste Anschlussvorrichtung 7 über vier Einzelanschlüsse, nämlich die Einzelanschlüsse 9, 10, 11 und 12, die durch jeweilige Stutzen ausgebildet sind. An diese Stutzen sind im endmontierten Zustand Schlauchleitungen für die Zuführung von Wasser angeschlossen. Es können eine Frischwasserleitung, eine Leitung für Warmwasser und zwei Leitungen für vollentsalztes Wasser vorgesehen sein.

Die zweite Anschlussvorrichtung 8 verfügt über einen deckelseitig ausgebildeten Anschlussstutzen 30. An diesen ist im endmontierten Zustand eine Abluftleitung angeschlossen, die an ein in den Figuren nicht näher dargestelltes Abluftaggregat strömungstechnisch angeschlossen ist.

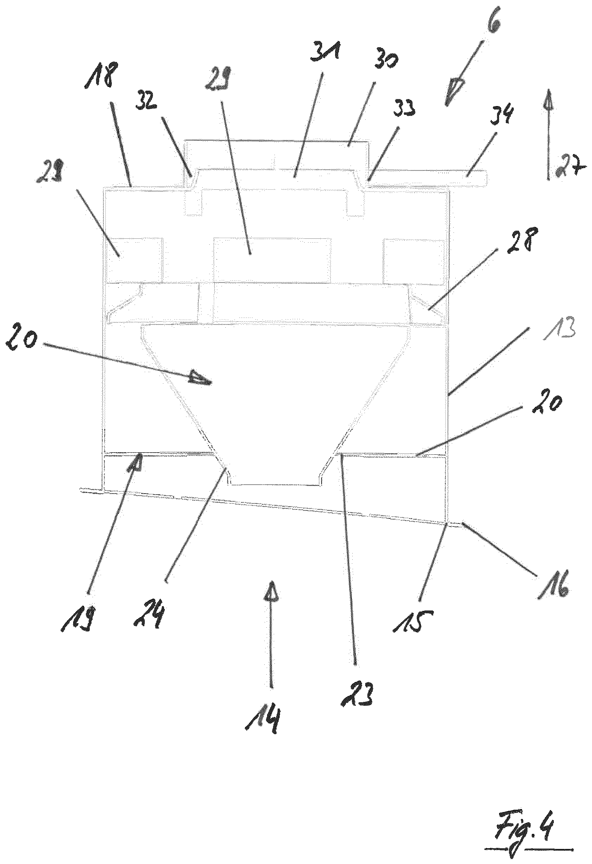

Wie sich aus einer weiteren Zusammenschau insbesondere der Figuren 3 und 4 ergibt, ist im Inneren des Gehäuses 13 ein Spritzschutz 19 ausgebildet. Dieser ist aus einem Scheibenelement 22 gebildet, das in Höhenrichtung 27 oberhalb der in der Spülbehälterwandung 4 ausgebildeten Öffnung 5 angeordnet ist. Dabei sind die Öffnung 5 und das Scheibenelement 22 derart aufeinander abgestimmt, dass eine Abschattung gegeben ist.

Das Scheibenelement 22 verfügt über eine Mittenausnehmung 23. Durch diese hindurch greift mit seinem spülraumseitigen Endabschnitt 24 ein trichterförmig ausgebildetes Fluidleitelement 20. Dabei verjüngt sich das Fluidleitelement 20 ausgehend von den Einzelanschlüssen 9 bis 12 in Richtung auf den spülbehälterseitigen Endabschnitt 24.

Wie insbesondere die Darstellung nach Fig. 4 erkennen lässt, ist das Fluidleitelement 20 im Durchmesser so bemessen, dass es unter Belassung eines Ringraums 26 beabstandet zur Wandung 25 des Gehäuses 13 angeordnet ist.

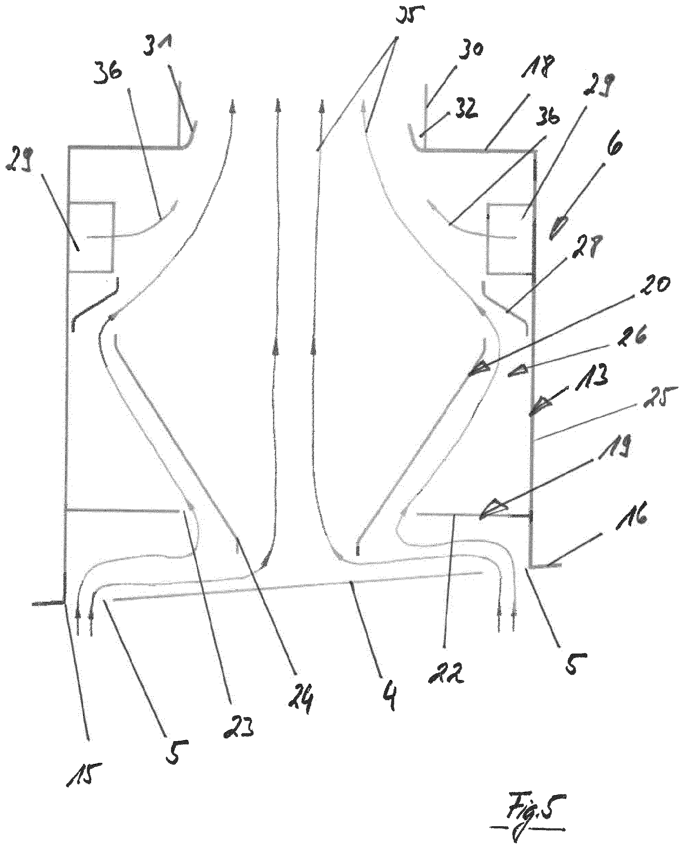

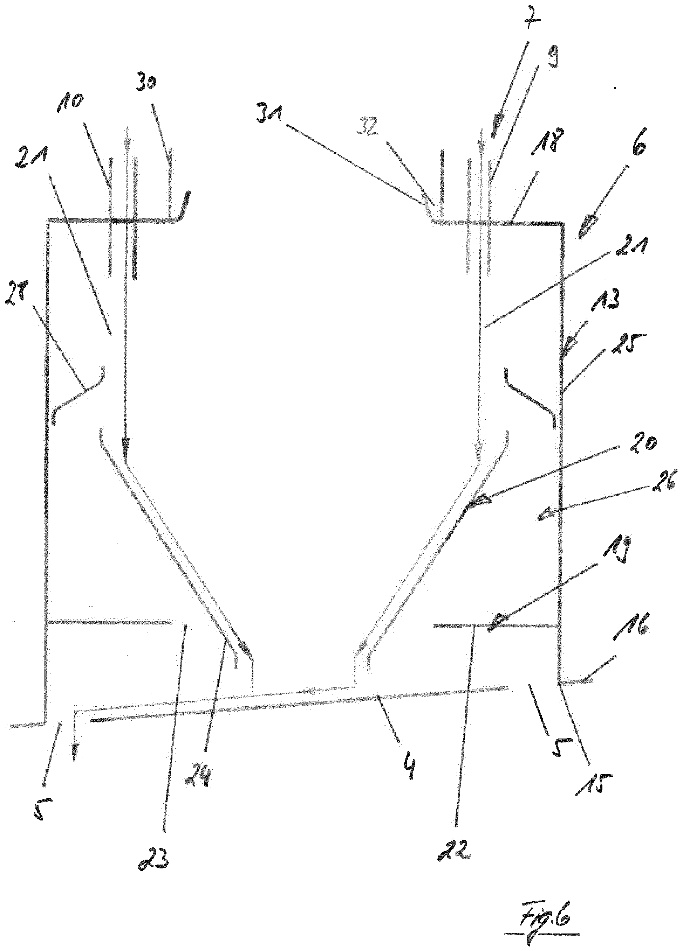

In Höhenrichtung 27 oberhalb des Fluidleitelementes 20 ist ein Ringteil 28 angeordnet, dass das Fluidleitelement 20 randseitig überragt, wie dies insbesondere die Darstellungen nach den Figuren 5 und 6 erkennen lassen.

Des Weiteren in Höhenrichtung 27 oberhalb des Ringteils 28 sind in der Gehäusewandung 25 des Gehäuses 13 Ausnehmungen in Form von Fenstern 29 angeordnet. Dabei ist die in Höhenrichtung 27 obere Fensterkante der Fenster 29 unterhalb des spülraumseitigen Endes der Einzelstutzen 9 bis 12 angeordnet. Dies lässt insbesondere die Darstellung nach den Figuren 3 und 4 erkennen.

Die Einzelanschlüsse 9 bis 12 ragen, wie insbesondere die Darstellung nach Fig. 6 erkennen lässt, nicht bis in den Bereich des Fluidleitelementes 20. Es ist vielmehr vorgesehen, dass die Einzelanschlüsse 9 bis 12 unter Zwischenschaltung einer jeweiligen Freifallstrecke 21 in das Fluidleitelement 20 einmünden, was ebenfalls der Darstellung nach Fig. 6 entnommen werden kann.

Die zweite Anschlussvorrichtung 8 verfügt über einen Anschlussstutzen 30, wie dies eine Zusammenschau der Figuren 3, 5 und 6 erkennen lässt. Dieser Anschlussstutzen 30 ist innenseitig mit einer umlaufenden Rinne 31 ausgerüstet, die einen Volumenraum 32 definiert. Dieser Volumenraum 32 ist an eine Ablauföffnung 33 strömungstechnisch angeschlossen (vgl. Fig. 3), an die sich ein Röhrchen 34 anschließt.

Die Fluiddurchströmungseinrichtung 6 dient einerseits dazu, Wasser als Spülflüssigkeit über die Öffnung 5 in den Spülraum 3 zu leiten sowie andererseits über die Öffnung 5 im Spülraum 3 entstandenen Dampf abzuführen. Die diesbezüglichen Funktionsweisen sind schematisch in den Figuren 5 und 6 dargestellt, wobei Fig. 5 den Abluftbetrieb und Fig. 6 den Wasserzuführbetrieb zeigen.

Im Abluftbetrieb, wie er in Fig. 5 dargestellt ist, wird über ein an den Anschlussstutzen 30 angeschlossenes Abluftaggregat ein Abluftstrom in Entsprechung der Pfeile 35 erzeugt. Infolge dessen kommt es zu einem Absaugen von Abluft aus dem Spülbehälter 2, wobei die Abluft die in der Spülbehälterwandung 4 ausgebildete Öffnung 5 passiert. Der Abluftstrom wird einerseits durch das Fluidleitelement 20 und andererseits an diesem vorbei geführt. Beide Strömungswege ergeben in Gänze einen Gesamtströmungsquerschnitt, der für eine gewünschte Menge an Abluftabfuhr geeignet ist.

Um einen zu hohen Abluftaustrag bei entsprechend hoher Betriebsleistung des angeschlossenen Abluftaggregats zu vermeiden, können über die Fenster 29 in Entsprechung der Pfeile 36 Frischluftanteile mit angesaugt werden. Es ist so sichergestellt, dass auch bei Abluftleistungen von z.B. über 250 m³/min trotzdem ein maximal beladener Spülraum 3 innerhalb der gewünschten Zeit auf die erforderliche Zieltemperatur von z.B. 93°C aufgeheizt werden kann.

Fig. 6 lässt den Wasserzulaufbetrieb erkennen. Über die in der Schnittdarstellung 6 zu erkennenden Anschlussstutzen 9 und 12 strömt Wasser in Entsprechung der Pfeile 37 ein. Dieses gelangt über die Freifallstrecken 21 zum Fluidleitelement 20. Dabei verhindert das Ringteil 28 ein Hochspritzen von Wasser bis zu den Fenstern 29.

Das in das Fluidleitelement 20 eingebrachte Wasser wird hier der Trichterform entsprechend kanalisiert und zum Endabschnitt 24 transportiert, von wo aus es dann auf die Spülbehälterwandung 4 abgegeben wird. Die Spülbehälterwandung 4 ist zur Horizontalen geneigt ausgerichtet, so dass das Wasser der Schwerkraft folgend mit Bezug auf die Zeichnungsebene nach Fig. 6 nach links entlang der Spülbehälterwandung 4 abströmt. Hier gelangt es dann zur ringförmigen Öffnung 5, die in der Spülbehälterwandung 4 ausgebildet ist, so dass das Wasser durch die Öffnung 5 hindurch in den Spülraum 3 gelangen kann.

Im bestimmungsgemäßen Spülbetrieb kommt es zu Spritzwasserbildung, nicht zuletzt durch sich drehende Sprüharme. Das Spritzwasser gelangt auch durch die Öffnung 5 hindurch. Durch die Verschattung der Öffnung 5 durch den Spritzschutz 19 wird aber verhindert, dass das Spritzwasser bis zu den Fenstern 29 gelangen kann. Es sammelt sich vielmehr nach einem Abprallen am Spritzschutz 19 wieder an der äußeren Oberfläche der Spülbehälterwandung 4 und kann über die Öffnung 5 zurück in den Spülraum 3 strömen.

### Bezugszeichen

- 1: Reinigungs- und/oder Desinfektionsautomat
- 2: Spülbehälter
- 3: Spülraum
- 4: Spülbehälterwandung
- 5: Öffnung
- 6: Fluiddurchströmungseinrichtung
- 7: erste Anschlussvorrichtung
- 8: zweite Anschlussvorrichtung
- 9: Einzelanschluss
- 10: Einzelanschluss
- 11: Einzelanschluss
- 12: Einzelanschluss
- 13: Gehäuse
- 14: Stirnseite
- 15: Ausnehmung
- 16: Flansch
- 17: Bohrung
- 18: Deckel
- 19: Spritzschutz
- 20: Fluidleitelement
- 21: Freifallstrecke
- 22: Scheibenelement
- 23: Mittenausnehmung
- 24: Endabschnitt
- 25: Gehäusewandung
- 26: Ringraum
- 27: Höhenrichtung
- 28: Ringteil
- 29: Fenster
- 30: Anschlussstutzen
- 31: Rinne
- 32: Volumenraum
- 33: Ablauföffnung
- 34: Röhrchen
- 35: Pfeil
- 36: Pfeil
- 37: Pfeil

## Patentansprüche

1. Reinigungs- und/oder Desinfektionsautomat mit einem einen Spülraum (3) bereitstellenden Spülbehälter (2), der eine Spülbehälterwandung (4) mit einer Öffnung (5) zur Beschickung des Spülraums (3) mit einem Fluid aufweist, und mit einer an die Öffnung (5) strömungstechnisch angeschlossenen Fluiddurchströmungseinrichtung (6), die eine erste Anschlussvorrichtung (7) für eine Fluidzufuhr in den Reinigungs- und/oder Desinfektionsautomaten und eine zweite Anschlussvorrichtung (8) für eine Fluidabfuhr aus dem Reinigungs- und/oder Desinfektionsautomaten aufweist, wobei die erste Anschlussvorrichtung (7) dazu dient, als Spülflüssigkeit vorgesehenes Wasser über die Fluiddurchströmungseinrichtung (6) durch die Öffnung (5) in den Spülraum (3) zu leiten und wobei die zweite Anschlussvorrichtung (8) dazu dient, ein aus dem Spülraum (3) durch die Öffnung (5) in die Fluiddurchströmungseinrichtung (6) geleitetes Luftgemisch aus dem Reinigungs- und/oder Desinfektionsautomaten abzuführen,
**dadurch gekennzeichnet, dass**
die Fluiddurchströmungseinrichtung (6) ein Gehäuse (13) aufweist, dessen spülbehälterseitige Stirnseite (14) für einen strömungstechnischen Anschluss an die Öffnung (5) in der Spülbehälterwandung (4) zumindest teilweise offen ausgebildet ist, wobei das Gehäuse (13)
einen mit der Öffnung (5) zusammenwirkenden Spritzschutz (19), der in Höhenrichtung (27) oberhalb der Öffnung (5) angeordnet ist,
und/oder
ein mit der ersten Anschlussvorrichtung (7) zusammenwirkendes Fluidleitelement (20), das nach Art eines Trichters ausgebildet ist und sich ausgehend von der ersten Anschlussvorrichtung (7) in Richtung auf die spülbehälterseitige Öffnung (5) konisch verjüngt,
aufweist.

2. Automat nach Anspruch 1,
**dadurch gekennzeichnet, dass**
die erste Anschlussvorrichtung (7) eine Mehrzahl von Einzelanschlüssen (9, 10, 11, 12) aufweist.

3. Automat nach Anspruch 1 oder 2,
**dadurch gekennzeichnet, dass**
das Gehäuse (13) kreiszylinderförmig ausgebildet ist.

4. Automat nach einem der vorhergehenden Ansprüche,
**dadurch gekennzeichnet, dass**
die erste Anschlussvorrichtung (7) oder die Einzelanschlüsse (9, 10, 11, 12) der ersten Anschlussvorrichtung (7) unter Zwischenschaltung einer jeweiligen Freifallstrecke (21) in das Fluidleitelement (20) einmündet bzw. einmünden.

5. Automat nach einem der vorhergehenden Ansprüche,
**dadurch gekennzeichnet, dass**
der Spritzschutz (19) ein mit einer Mittenausnehmung (23) ausgebildetes Scheibenelement (22) ist, wobei das Fluidleitelement (20) mit seinem spülbehälterseitigen Endabschnitt (24) die Mittenausnehmung (23) durchragt.

6. Automat nach Anspruch 5,
**dadurch gekennzeichnet, dass**
die Öffnung (5) in der Spülbehälterwandung (4) ringförmig ausgebildet ist und die Mittenausnehmung (23) des Spritzschutzes (19) umfangsseitig umgibt.

7. Automat nach einem der vorhergehenden Ansprüche,
**dadurch gekennzeichnet, dass**
das trichterförmig ausgebildete Fluidleitelement (20) unter Belassung eines Ringraums (26) beabstandet zur Wandung (25) des Gehäuses (13) angeordnet ist.

8. Automat nach Anspruch 7,
**dadurch gekennzeichnet, dass**
in Höhenrichtung (27) oberhalb des Ringraums (26) ein den Ringraum (26) abdeckendes Ringteil (28) angeordnet ist.

9. Automat nach einem der vorhergehenden Ansprüche 2 bis 8,
**dadurch gekennzeichnet, dass**
in Höhenrichtung (27) unterhalb der Einzelanschlüsse (9, 10, 11, 12) der ersten Anschlussvorrichtung (7) Fenster (29) in der Wandung (25) des Gehäuses (13) ausgebildet sind.

10. Automat nach einem der vorhergehenden Ansprüche,
**dadurch gekennzeichnet, dass**
die zweite Anschlussvorrichtung (8) einen Anschlussstutzen (30) aufweist, der koaxial zum Fluidleitelement (20) ausgerichtet ist.

11. Automat nach Anspruch 10,
**dadurch gekennzeichnet, dass**
der Anschlussstutzen (20) innenseitig eine umlaufende Rinne (21) aufweist.

12. Automat nach Anspruch 11,
**dadurch gekennzeichnet, dass**
der von der Rinne (31) bereitgestellte Volumenraum (32) an eine Ablauföffnung (33) strömungstechnisch angeschlossen ist.

## Claims

1. Cleaning and/or disinfection machine comprising a washing container (2) providing a washing chamber (3), which washing container comprises a washing container wall (4) having an opening (5) for loading the washing chamber (3) with a fluid, and having a fluid flow installation (6) fluidically connected to the opening (5), which fluid flow installation has a first connection device (7) for supplying fluid to the cleaning and/or disinfection machine and a second connection device (8) for removing fluid from the cleaning and/or disinfection machine, the first connection device (7) being used to guide water provided as flushing liquid via the fluid flow installation (6) through the opening (5) and into the washing chamber (3), and the second connection device (8) being used to discharge a mixture of air, which is guided out of the washing chamber (3) through the opening (5) and into the fluid flow installation (6), from the cleaning and/or disinfection machine,
**characterised in that**
the fluid flow installation (6) has a housing (13), the washing container-side end face (14) of which is at least partially open for a fluidic connection to the opening (5) in the washing container wall (4), the housing (13) having
a splash guard (19) which interacts with the opening (5) and is arranged above the opening (5) in the vertical direction (27),
and/or
a fluid conducting element (20) which interacts with the first connection device (7), is designed in the manner of a funnel and, starting from the first connection device (7), is conically tapered in the direction of the washing container-side opening (5).

2. Machine according to claim 1,
**characterised in that**
the first connection device (7) has a plurality of individual connections (9, 10, 11, 12).

3. Machine according to either claim 1 or claim 2,
**characterised in that**
the housing (13) is circular-cylindrical.

4. Machine according to any of the preceding claims,
**characterised in that**
the first connection device (7) or the individual connections (9, 10, 11, 12) of the first connection device (7) opens into or open into the fluid conducting element (20) with the interposition of a respective free-fall section (21).

5. Machine according to any of the preceding claims,
**characterised in that**
the splash guard (19) is a disc element (22) formed with a central recess (23), the fluid conducting element (20) projecting through the central recess (23) with its washing container-side end portion (24).

6. Machine according to claim 5,
**characterised in that**
the opening (5) in the washing container wall (4) is annular and surrounds the central recess (23) of the splash guard (19) circumferentially.

7. Machine according to any of the preceding claims,
**characterised in that**
the funnel-shaped fluid conducting element (20) is arranged at a distance from the wall (25) of the housing (13), thus leaving an annular space (26).

8. Machine according to claim 7,
**characterised in that**
a ring part (28) covering the annular space (26) is arranged above the annular space (26) in the vertical direction (27).

9. Machine according to any of the preceding claims 2 to 8,
**characterised in that**
windows (29) are formed in the wall (25) of the housing (13) below the individual connections (9, 10, 11, 12) of the first connection device (7) in the vertical direction (27).

10. Machine according to any of the preceding claims,
**characterised in that**
the second connection device (8) comprises a connection nozzle (30) which is oriented coaxially to the fluid conducting element (20).

11. Machine according to claim 10,
**characterised in that**
the connection nozzle (20) comprises a circumferential channel (21) on its inside.

12. Machine according to claim 11,
**characterised in that**
the volume space (32) provided by the channel (31) is fluidically connected to a drainage opening (33).

## Revendications

1. Machine automatique de nettoyage et/ou de désinfection, comportant un récipient de rinçage (2) fournissant un espace de rinçage (3), lequel récipient de rinçage présente une paroi de récipient de rinçage (4) comportant une ouverture (5) destinée à charger l'espace de rinçage (3) en fluide, et comportant un appareil d'écoulement de fluide (6) raccordé par communication fluidique à l'ouverture (5), lequel appareil d'écoulement de fluide présente un premier dispositif de raccordement (7) pour une alimentation en fluide dans la machine automatique de nettoyage et/ou de désinfection et un second dispositif de raccordement (8) pour une évacuation de fluide hors de la machine automatique de nettoyage et/ou de désinfection, le premier dispositif de raccordement (7) servant à guider de l'eau prévue comme liquide de rinçage, par l'intermédiaire de l'appareil d'écoulement de fluide (6), à travers l'ouverture (5) dans l'espace de rinçage (3), et le second dispositif de raccordement (8) servant à évacuer un mélange d'air, guidé hors de l'espace de rinçage (3) à travers l'ouverture (5) dans l'appareil d'écoulement de fluide (6), hors de la machine automatique de nettoyage et/ou de désinfection,
**caractérisée en ce que**
l'appareil d'écoulement de fluide (6) présente un boîtier (13) dont la face frontale (14) côté récipient de rinçage est réalisée au moins partiellement ouverte pour un raccordement par communication fluidique à l'ouverture (5) dans la paroi de récipient de rinçage (4), le boîtier (13) présentant
une protection contre les éclaboussures (19) coopérant avec l'ouverture (5), laquelle protection étant disposée dans la direction de la hauteur (27) au-dessus de l'ouverture (5),
et/ou
un élément de guidage de fluide (20) coopérant avec le premier dispositif de raccordement (7), lequel élément étant réalisé à la manière d'un entonnoir et se rétrécissant de manière conique à partir du premier dispositif de raccordement (7) en direction de l'ouverture (5) côté récipient de rinçage.

2. Machine automatique selon la revendication 1,
**caractérisée en ce que**
le premier dispositif de raccordement (7) présente une pluralité de raccordements individuels (9, 10, 11, 12).

3. Machine automatique selon la revendication 1 ou 2,
**caractérisée en ce que**
le boîtier (13) est réalisé en forme de cylindre à base circulaire.

4. Machine automatique selon l'une des revendications précédentes,
**caractérisée en ce que**
le premier dispositif de raccordement (7) ou les raccordements individuels (9, 10, 11, 12) du premier dispositif de raccordement (7) débouchent dans l'élément de guidage de fluide (20) avec intercalation d'une zone de chute libre (21) respective.

5. Machine automatique selon l'une des revendications précédentes,
**caractérisée en ce que**
la protection contre les éclaboussures (19) est un élément de disque (22) réalisé avec un évidement central (23), l'élément de guidage de fluide (20) faisant saillie à travers l'évidement central (23) avec sa section d'extrémité côté récipient de rinçage (24).

6. Machine automatique selon la revendication 5,
**caractérisée en ce que**
l'ouverture (5) dans la paroi de récipient de rinçage (4) est réalisée de manière annulaire et entoure l'évidement central (23) de la protection contre les éclaboussures (19) côté circonférence.

7. Machine automatique selon l'une des revendications précédentes,
**caractérisée en ce que**
l'élément de guidage de fluide (20) réalisé en forme d'entonnoir est disposé tout en laissant un espace annulaire (26) à une certaine distance de la paroi (25) du boîtier (13).

8. Machine automatique selon la revendication 7,
**caractérisée en ce que**
une pièce annulaire (28) recouvrant l'espace annulaire (26) est disposée, dans la direction de la hauteur (27), au-dessus de l'espace annulaire (26).

9. Machine automatique selon l'une des revendications précédentes 2 à 8,
**caractérisée en ce que**
des fenêtres (29) sont réalisées dans la paroi (25) du boîtier (13), dans la direction de la hauteur (27), en dessous des raccordements individuels (9, 10, 11, 12) du premier dispositif de raccordement (7).

10. Machine automatique selon l'une des revendications précédentes,
**caractérisée en ce que**
le second dispositif de raccordement (8) présente une tubulure de raccordement (30) qui est orientée coaxialement par rapport à l'élément de guidage de fluide (20).

11. Machine automatique selon la revendication 10,
**caractérisée en ce que**
la tubulure de raccordement (20) présente, côté interne, une rainure circonférentielle (21).

12. Machine automatique selon la revendication 11,
**caractérisée en ce que**
l'espace volumique (32) fourni par la rainure (31) est raccordé par communication fluidique à une ouverture d'évacuation (33).
